(19) Europäisches Patentamt
European Patent Office
Office européen des brevets

(11) EP 1 045 934 B1

(12) EUROPEAN PATENT SPECIFICATION

(45) Date of publication and mention of the grant of the patent:
**27.04.2005 Bulletin 2005/17**

(21) Application number: **98961084.5**

(22) Date of filing: **17.12.1998**

(51) Int Cl.$^7$: **D06P 5/08**, D06M 16/00, C11D 3/386, C11D 3/39, C12S 11/00

(86) International application number:
**PCT/DK1998/000560**

(87) International publication number:
**WO 1999/034054 (08.07.1999 Gazette 1999/27)**

(54) **PROCESS FOR REMOVAL OF EXCESS DYE FROM PRINTED OR DYED FABRIC OR YARN**

VERFAHREN ZUM ENTFERNEN VON ÜBERSCHÜSSIGEN FARBSTOFFEN VON BEDRUCKTEN ODER GEFÄRBTEN TEXTILIEN

PROCEDE D'ELIMINATION DE L'EXCEDENT DE TEINTURE DES TISSUS IMPRIMES OU TEINTES

(84) Designated Contracting States:
**AT BE CH CY DE DK ES FI FR GB GR IE IT LI LU MC NL PT SE**

(30) Priority: **23.12.1997 DK 152697**

(43) Date of publication of application:
**25.10.2000 Bulletin 2000/43**

(73) Proprietors:
- **Novozymes A/S**
  **2880 Bagsvaerd (DK)**
- **BAYER AG**
  **51368 Leverkusen (DE)**

(72) Inventors:
- **DAMHUS, Ture**
  **DK-2100 Copenhagen (DK)**
- **VOGT, Uwe**
  **D-40789 Monheim (DE)**

(56) References cited:
**WO-A1-92/18687**
**WO-A2-94/29425**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to a novel method of removing excess dye from newly printed or dyed fabric or yarn as well as a system and a composition for use in the method.

## BACKGROUND OF THE INVENTION

Printing and dyeing of textiles is carried out by applying dyes to the textile by any appropriate method for binding the dyestuff to the fibres in the textiles. Excess soluble dyestuff not bound to the fibres must be removed after dyeing to ensure fastness of the dyed textiles and to prevent unwanted dye transfer during laundering of the textiles by the consumer.

Generally, a large amount of water is required for complete removal of excess dye. In a conventional process the printed or dyed textile is first rinsed with cold water, then washed at high temperature with the addition of a suitable additive to decrease back-staining, like poly(vinylpyrrolidone) (PVP). The process is repeated until a satisfactory amount of dyestuff (and thickeners) has been removed. PVP can be added to reduce back-staining during rinsing, but this compound does not bleach the dye and is relatively expensive. Furthermore, the waste liquor from a conventional process tends to be strongly coloured and may represent a disposal problem, which is not reduced by the use of PVP.

WO 92/18687 discloses a method of bleaching excess dye from printed or dyed fabric by treating with a liquor containing an enzyme exhibiting peroxidase activity or oxidase activity, an $O_2$ or $H_2O_2$ source as applicable, and optionally an additional oxidizable substrate, such as a metal ion, a halide ion or an organic compound, such as a phenol.

However, the concentrations of such additional substrates necessary for enzymatically bleaching the excess dye in the rinse liquor may present a risk of bleaching the dyed textiles themselves.

Accordingly, it is an object of the present invention to provide a method for removing or bleaching excess dye without bleaching the dyed textile. This is achieved by a process disclosed in claim 1.

In the present invention the term "mediator" means an additional oxidizable substance improving the bleaching performance.

By suitable combination of mediator and enzyme it is possible to avoid bleaching of the dyed textile while bleaching dyes in solution, thereby reducing the amount of unbleached dye deposited on the fibres and thus increasing the wet fastness of the dyed or printed textile.

By this process it is furthermore possible to reduce the number of rinsing steps and the temperature of the rinsing water in the rinsing steps compared to the conventional processes, thereby saving energy and costs.

Another object of the present invention is a system disclosed in claim 16.

The components of the system may be combined as a solution, a slurry or granulates depending on the specific enzymes and mediators selected.

A further object of the present invention is the use of the components specified above for the preparation of a multi-component system for removal of excess dye or print from newly manufactured fabric or yarn.

## DETAILED DESCRIPTION OF THE INVENTION

### Fabric or yarn

The process of the invention is applicable to all types of textile materials, both natural fibres and synthetic fibres as well as blends thereof. Typical examples are cellulosic fibres (cotton and flax), modified cellulose fibres (e.g. acetate and triacetate), protein fibres (e.g. wool and silk), polyamide fibres (e.g. nylon 6 and 6,6), polyester fibres (e. g. poly(ethylene terephthalate)) and acrylic fibres.

The process of the invention may be applied to dyed yarn, to knitted, woven or non-woven fabric, or to garments made from dyed and/or printed fabric, especially garments made from differently coloured material.

### Printing method

The process of the invention is suited for excess dye bleaching after any kind of textile printing. Examples of commonly used techniques are printing on a Rotation film, a Rouleaux, a Flash film, or a Transfer film device. After printing the dye is fixed on the textile by e.g. steaming or treatment with hot air.

### Dyeing method

The process of the invention is suited for excess dye bleaching after any kind of dyeing. The dyeing of textiles is for example carried out by passing the fabric through a concentrated solution of dye, followed by storage of the wet

fabric in a vapour tight enclosure to permit time for diffusion and reaction of the dye with the fabric substrate prior to rinsing off un-reacted dye. Alternatively, the dye may be fixed by subsequent steaming of the textile prior to rinsing.

The process applies to any kind of dyes, such as reactive dyes.

Enzyme

Enzymes exhibiting peroxidase activity or laccase activity are those which by using hydrogen peroxide or molecular oxygen, respectively are capable of oxidising a variety of compounds, such as phenols and aromatic amines.

According to the invention the concentration of enzyme is 0.005 to 5 mg enzyme protein per 1 of rinse liquor, preferably, 0.02 to 2 mg enzyme protein per 1 of rinse liquor, more preferably 0.05 to 1 mg enzyme protein per 1 of rinse liquor. According to the liquor ratio, this may be translated to dosages of enzyme per kg of fabric, e.g. at a liquor ratio of 10:1, the most preferred enzyme dosage is from 0.5 to 10 mg enzyme per kg of textile fabric.

Peroxidase activity exhibiting enzymes

An enzyme exhibiting peroxidase activity may be any peroxidase comprised by the enzyme classification (EC 1.11.1.7), or a haloperoxidase, such as a chloride peroxidase (EC 1.11.1.10) or any fragment or synthetic or semisynthetic derivatives thereof exhibiting enzymatic activity (e.g. porphyrin ring systems or microperoxidases, cf. e.g. US 4,077,768, EP 537 381, WO 91/05858 and WO 92/16634). Such enzymes are known from microbial, plant and animal origins.

Preferably, the peroxidase employed in the method of the invention is producible by planes (e.g. horseradish or soybean peroxidase), in particular soybean peroxidase, or by microorganisms, such as fungi (including filamentous fungi and yeasts) or bacteria.

Some preferred fungi include strains belonging to the subdivision *Deuteromycotina,* class *Hyphomycetes*, e. g., *Fusarium, Humicola, Tricoderma, Myrothecium, Verticillum, Arthromyces, Caldariomyces, Ulocladium, Embellisia, Cladosporium* or *Dreschlera,* in particular *Fusarium oxysporum* (DSM 2672), *Humicola insolens, Trichoderma resii, myrothecium* verrucana (IFO 6113), *Verticillum alboatrum, Verticillum dahlie, Arthromyces ramosus* (FERM P-7754), *Caldariomyces fumago, Ulocladium chartarum, Embellisia alli* or *Dreschlera halodes*.

Other preferred fungi include strains belonging to the subdivision *Basidiomycotina,* class *Basidiomycetes,* e. g. *Coprinus, Phanerochaete, Coriolus* or *Trametes,* in particular *Coprinus cinereus* f. *microsporus* (IFO 8371), *Coprinus macrorhizus, Phanerochaete chrysosporium* (e.g. NA-12) or *Trametes* (some classes previously called *Polyporus* have been renamed to *Trametes*), e.g., *T. versicolor* (e.g. PR4 28-A).

Further preferred fungi include strains belonging to the subdivision Zygomycotina, class Mycoraceae, e.g. Rhizopus or Mu*cor,* in particular *Mucor hiemalis.*

Some preferred bacteria include strains of the order *Actinomycetales*, e.g., *Streptomyces spheroides* (ATTC 23965), *Streptomyces thermoviolaceus* (IFO 12382) or *Streptoverticillum verticillium* ssp. *verticillium*.

Other preferred bacteria include *Bacillus pumilus* (ATCC 12905), *Bacillus stearothermophilus, Rhodobacter sphaeroides, Rhodomonas palustri, Streptococcus lactis, Pseudomonas purrocinia* (ATCC 15958) or *Pseudomonas fluorescens* (NRRL B-11) .

Further preferred bacteria include strains belonging to *Myxococcus,* e.g., *M. virescens*.

The peroxidase may furthermore be one which is producible by a method comprising cultivating a host cell transformed with a recombinant DNA vector which carries a DNA sequence encoding said peroxidase as well as DNA sequences encoding functions permitting the expression of the DNA sequence encoding the peroxidase, in a culture medium under conditions permitting the expression of the peroxidase, and recovering the peroxidase from the culture.

Particularly, a recombinantly produced peroxidase is a peroxidase derived from a *Coprinus sp.,* in particular *C. macrorhiz*us or *C. cinereus* according to WO 92/16634, or a variant thereof.

In the context of this invention, peroxidase acting compounds comprise peroxidase active fragments derived from cytochromes, hemoglobin or peroxidase enzymes, and synthetic or semisynthetic derivatives thereof, e.g. iron complexes of porphyrin or phthalocyanine and derivatives thereof.

Laccase and laccase related enzymes

In the context of this invention, the term "enzymes exhibiting laccase activity" means laccases and laccase related enzymes, such as any laccase comprised by the enzyme classification (EC 1.10.3.2), any catechol oxidase comprised by the enzyme classification (EC 1.10.3.1), any bilirubin oxidase comprised by the enzyme classification (EC 1.3.3.5) or any monophenol mono-oxygenase comprised by the enzyme classification (EC 1.14.99.1).

The laccases are known from microbial and plant origin. The microbial laccases may be derived from bacteria or fungi (including filamentous fungi and yeasts) and suitable examples include a laccase derivable from a strain of

*Aspergillus, Neurospora,* e.g., *N. crassa, Podospora, Botrytis, Collybia, Fomes, Lentinus, Pleurotus, Trametes,* e.g., *T. villosa* and *T. versicolor, Rhizoctonia*, e.g., *R. solani, Coprinus,* e.g. *C. plicatilis* and *C. cinereus, Psatyrella, Myceliophthora,* e.g. *M. thermophila, Schytalidium, Polyporus,* e.g., *P. pinsitus, Phlebia,* e.g., *P. radiata* (WO 92/01046), or *Coriolus,* e.g., *C. hirsutus* (JP 2-238885), in particular a laccase derivable from a strain of *Fomes, Trametes, Rhizoctonia, Coprinus, Myceliophthora, Schytalidium,* or *Polyporus*.

The laccase or the laccase related enzyme may furthermore be one which is producible by a method comprising cultivating a host cell transformed with a recombinant DNA vector which carries a DNA sequence encoding said laccase as well as DNA sequences encoding functions permitting the expression of the DNA sequence encoding the laccase, in a culture medium under conditions permitting the expression of the laccase, and recovering the laccase from the culture.

Oxidation agent

If the oxidizing enzyme requires a source of hydrogen peroxide, the source may be hydrogen peroxide or a hydrogen peroxide precursor for in situ production of hydrogen peroxide, e.g., a percarbonate or a perborate, a persulfate, such as a tri-oxo(peroxo)sulfate or a μ-peroxo-bis(trioxosulfate), a hydrogen peroxide-urea addition compound, a peroxycarboxylic acid or a salt thereof or a hydrogen peroxide generating enzyme system, e.g., an oxidase and a substrate for the oxidase, e.g. an amino acid oxidase and a suitable amino acid.

Hydrogen peroxide may be added at the beginning of or during the process, e.g., in a concentration corresponding to 0.01-50 mM $H_2O_2$, preferably 0.1 to 5 mM.

If the oxidizing enzyme requires molecular oxygen, molecular oxygen from the atmosphere will usually be present in sufficient quantity. Otherwise pure $O_2$ may be led to the rinse liquor, or an $O_2$ generating enzymatic system, e.g. a system based on hydrogen peroxide and a catalase, may be added.

Mediator

According to the invention at least one mediator is added to the rinse liquor. The mediator is a compound of the general formula I:

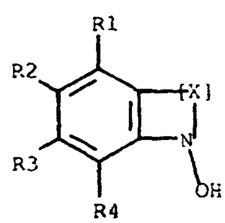

wherein $R^1$, $R^2$, $R^3$, $R^4$ are individually selected from the group consisting of hydrogen, halogen, hydroxy, formyl, carboxy and salts and esters thereof, amino, nitro, $C_1$-$C_{12}$ alkyl, $C_1$-$C_6$ alkoxy, carbonyl($C_1$-$C_{12}$ alkyl), aryl, in particular phenyl, sulfo, aminosulfonyl, carbamoyl, phosphono, phosphonooxy, and salts and esters thereof, wherein the $R^1$, $R^2$, $R^3$, $R^4$ may be substituted with $R^5$, wherein $R^5$ represents hydrogen, halogen, hydroxy, formyl, carboxy and salts and esters thereof, amino, nitro, $C_1$-$C_{12}$ alkyl, $C_1$-$C_6$ alkoxy, carbonyl($C_1$-$C_{12}$ alkyl), aryl, in particular phenyl, sulfo, aminosulfonyl, carbamoyl, phosphono, phosphonooxy, and salts and esters thereof,
[X] represents a group from (-N=N-), $(-N=CR^6-)_m$, $(-CR^6=N-)_m$, $(-CR^7=CR^6-)_m$,
and m is 1 or 2.
In a more preferred embodiment of the invention the mediator is a compound of the general formula II:

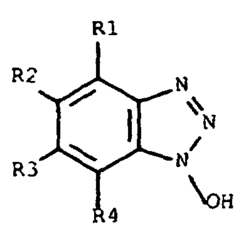

wherein $R^1$, $R^2$, $R^3$, $R^4$ are individually selected from the group consisting of hydrogen, halogen, hydroxy, formyl, carboxy and salts and esters thereof, amino, nitro, $C_1$-$C_{12}$ alkyl, $C_1$-$C_6$ alkoxy, carbonyl($C_1$-$C_{12}$ alkyl), aryl, in particular phenyl, sulfo, aminosulfonyl, carbamoyl, phosphono, phosphonooxy, and salts and esters thereof, wherein the $R^1$, $R^2$, $R^3$, $R^4$ may be substituted with $R^5$, wherein $R^5$ represents hydrogen, halogen, hydroxy, formyl, carboxy and salts and esters thereof, amino, nitro, $C_1$-$C_{12}$ alkyl, $C_1$-$C_6$ alkoxy, carbonyl ($C_1$-$C_{12}$ alkyl), aryl, in particular phenyl, sulfo, aminosulfonyl, carbamoyl, phosphono, phosphonooxy, and salts and esters thereof.

The mediator may also be a salt or an ester of formula I or II.

Preferred mediators are selected from the group consisting of 1-hydroxybenzotriazole; 1-hydroxybenzotriazole hydrate; 1-hydroxybenzotriazole sodium salt; 1-hydroxybenzotriazole potassium salt; 1-hydroxybenzotriazole lithium salt; 1-hydroxybenzotriazole ammonium salt; 1-hydroxybenzotriazole calcium salt; 1-hydroxybenzotriazole magnesium salt; and 1-hydroxybenzotriazole-6-sulphonic acid.

A particularly preferred mediator is 1-hydroxybenzotriazole.

All the specifications of N-hydroxy compounds above are understood to include tautomeric forms such as N-oxides whenever relevant.

Usually, the concentration of mediator in the rinse liquor is 1 µM to 1 mM, more preferably 10 µM to 0.5 mM.

## Additives

The rinse liquor may comprise further additives, such as surfactants and/or water conditioning agents.

## Multi-component system

In order to carry out the process described above a multi-component system is added to the rinse liquor in at least one of the rinsing steps.

The components of the multi-component system may individually be in one of several product forms, such as a slurry, a solution or a granulate.

In one embodiment of the invention two components are mixed in the represented form, such as a co-granulate, a solution or a slurry comprising enzyme and mediator.

In cases of co-granulates, the co-granulate may comprise at least one enzyme and at least one mediator. Another example of a co-granulate is a granulate comprising at least two different enzymes and optionally at least one mediator.

In a further embodiment the system is a mixture of granulates wherein the component(s) in one granulate is (are) enzyme(s) and the component(s) in another granulate is(are) mediatorts).

According to the present invention a preferred multi-component system disclosed in claim 16 is used.

The enzymes exhibiting peroxidase activity or laccase activity are preferably as described above.

The system may comprise an oxidation agent, but in cases where the enzyme is an enzyme exhibiting laccase activity, molecular oxygen from the atmosphere is normally sufficient, and the system used will not comprise an oxidation agent. However, when the enzymes used require addition of an oxidation agent those are as described above. In all cases wherein a $H_2O_2$ source is the oxidation agent the enzyme and oxidation agent may not be mixed before use.

The mediator is a compound of the general formula I as described above,

A particularly preferred mediator is 1-hydroxybenzotriazole.

All the specifications of N-hydroxy compounds above are understood to include tautomeric forms such as N-oxides whenever relevant.

A further aspect of the present invention is the use of components as disclosed in claim 31 for the preparation of a multi-component system for removal of excess dye or print from newly manufactured fabric or yarn.

## Process conditions

The removal of excess dye, according to the invention, may comprise rinsing with rinse liquor in 2 to 6 rinsing steps, more preferred in 2 to 5 rinsing steps, even more preferred in 2 to 4 rinsing steps, in particular in 3 to 4 rinsing steps. The amount of rinsing steps is dependent on the concentration of the mediator and of the concentration of the peroxidase.

The multi-component system as defined according to this invention may be used in any of the rinsing steps performed, however it is preferably added in one of the last rinsing steps, in particular in the third or fourth rinsing step.

The process may be run in batch mode or continuous mode. The process may be applied on a winch, a beck, a jet dyer, an open-width washing machine, a J or U box, a steamer, or any other equipment available suitable for a rinsing process.

The process conditions must be chosen according to the characteristics of the enzyme in question. The temperature at the rinsing step comprising a multi-component system as defined above is preferably ranging from 40°C to 80°C, such as from 50°C to 70°C, and pH is typically in the range of 5.5-9.5, such as 6.5-9.

Fatness

Fatsness (wet, crock, light, etc.) may be measured by various methods as known in the art. Wet fastness may be measured as described below. Colourfastness to crocking, which is designed to determine the amount of colour transferred from the surface of coloured materials to other surfaces by rubbing, may be measured according to AATCC Test Method 8-1996. Colourfastness to light, in which samples of the material to be tested and the agreed upon comparison standard(s) are exposed simultaneously to a light source under specified conditions, may be measured according to AATCC Test Method 16-1993.

Wet fastness

The multi-component system as defined above is added to the rinsing liquor to prevent re-deposition of solubilised excess dye by bleaching it in solution.

The wet fastness or water fastness reflects the degree to which this has successfully been achieved.

In the present invention the wet fastness is measured by the standard method (DIN 54 006). Briefly, the method comprises soaking a dyed fabric and pressing it together with swatches of white fabric. After separate drying of the fabrics, the swatches are evaluated for staining.

The degree of wet fastness is indicated on a scale, the higher number the better wet fastness. 1 means very low wet fastness, whereas 5 means very high wet fastness.

Colour measurement (Example 5)

A Gretag-Mecbath Colour Eye 3100 was used according to the manufacturer's instructions to evaluate the chromaticity using the change in the colour space coordinates L*a*b* (CIELAB-system), where as usual:

L* gives the change in white/black on a scale from 0 to 100, and a decrease in L* means an increase in black colour (decrease in white colour) and an increase in L* means an increase in white colour (decrease in black colour).

a* gives the change in red/green, and a decrease in a* means an increase in green colour (decrease in red colour), and an increase in a* means an increase in red colour (decrease in green colour).

b* gives the change in blue/yellow, and a decrease in b* means an increase in blue colour (decrease in yellow colour), and an increase in b* means an increase in yellow colour (decrease in blue colour) (Vide WO 96/12846 NOVO).

The Gretag-Macbeth Colour Eye 3100 was operated in the L*a*b* colour space. The light source was D65 standard light. The software used for evaluation was Optiview Quality Control 1.7c. The observation angle was 10°. The instrument was calibrated using a Macbeth calibration plate (white). Each result was an average of 10 measurements. Fabric rinsed without enzyme and mediator was measured and the coordinates L*a*b* were calculated and entered as a reference. The coordinates of the samples were then for each of L*, a*, b* calculated as the difference (Δ) of the average of the measurements on each swatch from the reference value.

The present invention is further illustrated in the following examples which are not in any way intended to limit the scope of the invention as claimed.

**Example 1**

Reactive dyeing of cotton fabric followed by an enzymatic rinsing process

Knitted, bleached 100 % cotton was dyed in a Mathis jet-dyer (laboratory scale jet dyeing machine) under the following conditions:

| | |
|---|---|
| Water | softened water, 10 1/kg of fabric |
| Temperature | 50°C |
| Dyestuff | 4 % LEVAFIX Scarlet E-2GA (Reactive Red 123) |
| $Na_2SO_4$ | 50 g/l |
| $Na_2CO_3$ | 4 g/l |
| NaOH (32 %) | 2 ml/l |
| LEVEGAL RL | 1.0 g/l (levelling agent) |

(continued)

| | |
|---|---|
| ERKANTOL NR | 1.0 g/l (wetting agent) |
| PERSOFTAL L | 1.0 g/l (crease-preventing agent) |
| RESPUMIT S | 1.0 g/l (antifoaming agent) |

LEVAFIX Scarlett E-2GA is a product of DyStar.

LEVEGAL RL, ERKANTOL NR, PERSOFTAL L and RESPUMIT S are products of BAYER.

The dyeing process started at 50°C by addition of dyestuff, $Na_2SO_4$, LEVEGAL RL, ERKANTOL NR, PERSOFTAL L and RESPUMIT S. $Na_2CO_3$ was added 30 minutes after start and NaOH 60 minutes after start. During the whole process the temperature was held at 50°C. 60 minutes after addition of NaOH the dyeing process was stopped by draining off the dyeing liquor, whereafter the rinsing process was started.

The rinsing process was carried out as follows:

*First rinsing step:*

Addition of fresh softened water; 10 1/kg fabric.
Rinsing 10 minutes at 40°C.
Draining the rinsing liquor.

*Second rinsing step:*

Addition of fresh softened water; 10 1/kg fabric.
Addition of 10 ml/l of acetic acid (6 % solution in water)
Rinsing 20 minutes at 95°C.
Draining the rinsing liquor.

*Third rinsing step:*

Addition of fresh softened water; 10 l/kg fabric.
Addition of 5.0 ml/l of potassium phosphate buffer (1.0 M, pH=7.0)
Rinsing 40 minutes at 60°C.
Addition of 0.8 mg/l Peroxidase SP502, 55 mg/l (0.4 mM) HOBT and 39 mg/l (0.4 mM) $H_2O_2$ (35 % solution in water)
Rinsing 10 minutes at 60°C.
Draining the rinsing liquor.

SP502 was a liquid preparation of recombinant *Coprinus cinereus* peroxidase supplied by Novo Nordisk A/S (produced as described in WO 92/16634). HOBT was 1-hydroxybenzotriazole ex Sigma.

The fabric was squeezed and dried. The wet fastness was determined according to DIN 54 006. The degree of fastness was found to be 3 (adjacent fabric cotton).

**Example 2 (for comparison)**

<u>Conventional 3 step rinsing process</u>

The dyeing process was carried out as described in Example 1. The rinsing steps were carried out as follows.

*First rinsing step:*

Addition of fresh softened water; 10 1/kg fabric.
Rinsing 10 minutes at 40°C.
Draining the rinsing liquor.

*Second rinsing step:*

Addition of fresh softened water; 10 1/kg fabric.

Rinsing 20 minutes at 95°C.
Draining the rinsing liquor.

*Third rinsing step:*

Addition of fresh softened water; 10 1/kg fabric.
Rinsing 10 minutes at 80°C.
Draining the rinsing liquor.

The fabric was squeezed and dried. The wet fastness was determined according to DIN 54 006. The degree of fastness was found to be 2 (adjacent fabric cotton).

**Example 3 (for comparison)**

Conventional 4 step rinsing process

The dyeing process was carried out as described in Example 1. The rinsing steps were carried out as follows.

*First rinsing step:*

Addition of fresh softened water; 10 l/kg fabric.
Rinsing 10 minutes at 40°C.
Draining the rinsing liquor.

*Second rinsing step:*

Addition of fresh softened water; 10 l/kg fabric.
Rinsing 10 minutes at 70°C.
Draining the rinsing liquor.

*Third rinsing step:*

Addition of fresh softened water; 10 1/kg fabric.
Rinsing 20 minutes at 95°C.
Draining the rinsing liquor.

*Fourth rinsing step:*

Addition of fresh softened water; 10 1/kg fabric.
Rinsing 10 minutes at 40°C.
Draining the rinsing liquor.

The fabric was squeezed and dried. The wet fastness was determined according to DIN 54 006. The degree of fastness was found to be 2-3 (adjacent fabric cotton).

**Example 4 (for comparison)**

Conventional 6 step rinsing process

The dyeing process was carried out as described in Example 1. The rinsing steps were carried out as follows.

*First rinsing step:*

Addition of fresh softened water; 10 1/kg fabric.
Rinsing 10 minutes at 40°C.
Draining the rinsing liquor.

*Second rinsing step:*

Addition of fresh softened water; 10 1/kg fabric.
Rinsing 10 minutes at 70°C.
Draining the rinsing liquor.

*Third rinsing step:*

Addition of fresh softened water; 10 1/kg fabric.
Rinsing 10 minutes at 70°C.
Draining the rinsing liquor.

*Fourth rinsing step:*

Addition of fresh softened water; 10 1/kg fabric.
Rinsing 20 minutes at 95°C.
Draining the rinsing liquor.

*Fifth rinsing step:*

Addition of fresh softened water; 10 1/kg fabric.
Rinsing 10 minutes at 70°C.
Draining the rinsing liquor.

*Sixth rinsing step:*

Addition of fresh softened water; 10 1/kg fabric.
Rinsing 10 minutes at 40°C.
Draining the rinsing liquor.

The fabric was squeezed and dried. The wet fastness was determined according to DIN 54006. The degree of fastness was found to be 3 (adjacent fabric cotton).

Conclusion: The wet fastness 3 (corresponding to the conventional 6 step rinsing process) was also obtained by the process according to the invention (see Example 1), whereby a substantial amount of rinsing steps can be avoided (saving water and energy and process time).

**Example 5**

Reactive dyeing of cotton fabric followed by enzymatic rinsing processes using two different mediators

Knitted, bleached 100 % cotton was dyed in a Mathis jet-dyer (laboratory scale jet dyeing machine) at the following conditions:

| | |
|---|---|
| Water | softened water, 10 1/kg of fabric |
| Dyestuff | 1 % Remazol Yellow RR |
| | 1 % Remazol Red RR |
| | 1 % Remazol Blue RR |
| $Na_2SO_4$ | 70 g/l |
| $Na_2CO_3$ | 5 g/l |
| NaOH (32 % in water) | 3 ml/l |

| | | |
|---|---|---|
| LEVEGAL RL | 1.0 g/l | (levelling agent) |
| ERKANTOL NR | 1.0 g/l | (wetting agent) |
| PERSOFTAL L | 1.0 g/l | (crease-preventing agent) |

Remazol Yellow RR, Remazol Red RR, and Remazol Blue RR are products of DyStar.

LEVEGAL RL, ERKANTOL NR, and PERSOFTAL L are products of BAYER.

The dyeing process started at 30°C by addition of $Na_2CO_3$, NaOH (1 ml/l), $Na_2SO_4$, LEVEGAL RL, ERKANTOL NR, PERSOFTAL L. The dyestuffs were added 15 minutes after start. 30 minutes after start the temperature was increased up to 50°C within 20 minutes. 30 minutes after reaching 50°C the remaining NaOH (2 ml/l) was added. 45 minutes after the addition of NaOH (2 ml/l) the dyeing process was finished by draining off the dyeing liquor, whereafter the rinsing process was started.
The following rinsing process was carried out for each mediator (1-hydroxybenzotriazole (according to the invention); and methyl syringate (comparison)):

*First rinsing step:*

Addition of fresh softened water; 10 1/kg fabric.
Rinsing 10 minutes at 40°C.
Draining the rinsing liquor.

*Second rinsing step:*

Addition of fresh softened water; 10 1/kg fabric.
Addition of 10 ml/l of acetic acid (6 % solution in water)
Rinsing 20 minutes at 95°C.
Draining the rinsing liquor.

*Third rinsing step:*

Addition of fresh softened water; 10 l/kg fabric.
Addition of 5.0 ml/l of potassium phosphate buffer (1.0 M, pH=7.0)
Rinsing 40 minutes at 60°C.
Addition of 0.8 mg/l Peroxidase SP502, 0.4 mM mediator and 39 mg/l (0.4 mM) $H_2O_2$ (35 % solution in water)
Rinsing 10 minutes at 60°C.
Draining the rinsing liquor.

SP502 was a liquid preparation of recombinant *Coprinus cinereus* peroxidase supplied by Novo Nordisk A/S.
The fabric was squeezed and dried. A colour measurement was performed using a colour measuring instrument (Gretag-Macbeth Colour Eye 3100) as described above.

Results:

Mediator: 1-hydroxybenzotriazole

$$\Delta L^* = 0.756$$

$$\Delta a^* = 0.045$$

$$\Delta b^* = 0.022$$

Mediator: Methyl syringate

$$\Delta L^* = 3.142$$

$$\Delta a^* = 0.134$$

$$\Delta b^* = 0.834$$

Conclusion: It can be seen that methyl syringate has a significant and undesired influence on the chromaticity of the fabric. The positive Δb* of 0.834 indicates a very strong and not acceptable increase in yellow colour whereas a Δb* of 0.022 which is found for 1-hydroxybenzotriazole is negligible and not visible. It is seen that also ΔL* and Δa* are significantly smaller in the treatment according to the invention than in the comparison treatment.

**Example 6**

Enzymatic bleaching of soluble dyes

The dyes tested were Reactive Black 5 (Remazol Black B), Reactive Red 198 (Remazol Red RB), Reactive Blue 220(Remazol Brilliant Blue BB), Reactive Blue 21 (Remazol Turquoise Blue G), and Reactive Orange 107 (Remazol Golden Yellow RNL), all ex Dystar. All dyes were dissolved in a 0.5 mM sodium phosphate buffer (pH 7.0) to an initial absorbance of approximately 0.4 at the wavelength λmax of maximum absorbance within the visible range. The solutions were then placed in a thermostated quartz cell in a HP 8453 diode array spectrophotometer, the three components of the enzymatic oxidation system were added (CiP to 0.2 mg/l, HOBT to 100 μM, hydrogen peroxide to 200 μM), and the absorbance ABS(λmax) at λmax monitored over time. The degree of bleaching at 5 min, i.e. the decrease in ABS(λmax) over 5 min divided by ABS(λmax) at t = 0, is shown below, measured at three temperatures.

Degree of bleaching at 5 min(%):

Reactive Black 5 (λmax=596 nm) :
79 (60°C); 82 (70°C); 72 (80°C);
Reactive Red 198 (λmax=517 nm):
97 (60°C); 100 (70°C); 88 (80°C);
Reactive Blue 220 (λmax=608 nm):
100 (60°C); 98 (70°C); 30 (80°C);
Reactive Blue 21 (λmax=663 nm):
100 (60°C) ; 88 (70°C); 61 (80°C);
Reactive Orange 107 (λmax=408 nm):
90 (60°C) ; 67 (70°C) ; 36 (80°C).

This example demonstrates that one of the preferred mediators according to the invention, HOBT (1-hydroxybenzotriazole), combined with *Coprinus cinereus* peroxidase (CiP) and hydrogen peroxide, provides high degrees of bleaching of soluble dyes in short time with a range of reactive dyes.

**Claims**

**1.** A process for removal of excess dye from newly manufactured printed or dyed fabric or yam, comprising treatment with a rinse liquor comprising

- at least one enzyme selected from the group consisting of enzymes exhibiting peroxidase activity or laccase activity,
- an oxidation agent, and
- at least one mediator wherein the mediator is a compound of the general formula I:

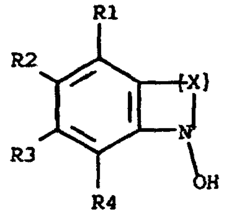

wherein $R^1$, $R^2$, $R^3$, $R^4$ are individually selected from the group consisting of hydrogen, halogen, hydroxy, formyl, carboxy and salts and esters thereof, amino, nitro, $C_1$-$C_{12}$ alkyl, $C_1$-$C_6$ alkoxy, carbonyl($C_1$-$C_{12}$ alkyl), aryl, in particular phenyl, sulfo, aminosulfonyl, carbamoyl, phosphono, phosphonooxy, and salts and esters thereof, wherein the $R^1$, $R^2$, $R^3$, $R^4$ may be substituted with $R^5$, wherein $R^5$ represents hydrogen, halogen, hydroxy, formyl, carboxy and salts and esters thereof, amino, nitro, $C_1$-$C_{12}$ alkyl, $C_1$-$C_6$ alkoxy, carbonyl($C_1$-$C_{12}$ alkyl), aryl, in particular phenyl, sulfo, aminosutfonyl, carbamoyl, phosphono, phosphonooxy, and salts and esters thereof,
[X] represents a group from (-N=N-), $(\text{-N=CR}^6\text{-})_m$, $(\text{-CR}^6\text{=N-})_m$, $(\text{-CR}^7\text{=CR}^8\text{-})_m$,
and m is 1 or 2, wherein said mediator is present in said rinse liquor at a concentration of from 1 µM to 1 mM, and

- optionally additives.

**2.** A process according to claim 1, wherein the enzyme is a laccase (EC 1.10.3.2), a catechol oxidase (EC 1.10.3.1). a bilirubin oxidase (EC 1.3.3.5), a peroxidase (EC 1.11.1.7). or a haloperoxidase, such as a chloride peroxidase (EC 1.11.1.10) or any fragment derived therefrom exhibiting enzymatic activity or synthetic or semisynthetic derivatives thereof.

**3.** A process according to claim 2, wherein the peroxidase is derived from a strain of *Coprinus* or from soybean.

**4.** A process according to claim 2, wherein the laccase is derived from a strain of *Fomes, Trametes, Rhizoctonia, Coprinus, Myceliophthora, Schytalidium,* or *Polyporus*.

**5.** A process according to any of the preceding claims, wherein the amount of enzyme is 0.005 to 5 mg enzyme protein per l of rinse liquor, preferably. 0.02 to 2 mg enzyme protein per l of rinse liquor, more preferably 0.05 to 1 mg enzyme protein per l of rinse liquor.

**6.** A process according to any of the preceding claims, wherein the oxidation agent is a $H_2O_2$ source.

**7.** A process according to claim 6, wherein the $H_2O_2$ source is hydrogen peroxide, a perborate, a percarbonate, a persulfate, a peroxycarboxylic acid or a salt thereof, or an enzymatic system capable of generating hydrogen peroxide.

**8.** A process according to claim 7, wherein the concentration of $H_2O_2$ is from 0.01 to 50 mM, preferably 0.1 to 5 mM.

**9.** A process according to any of the claims 1-5, wherein the oxidation agent is a $O_2$ source.

**10.** A process according to claim 9, wherein the $O_2$ source is air, pure $O_2$, or an $O_2$ generating enzymatic system.

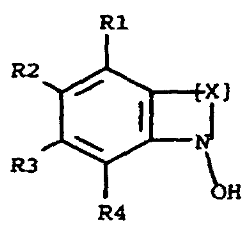

**11.** A process according to claim 1, wherein the mediator is a compound of the general formula II:

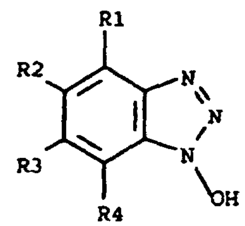

wherein $R^1$, $R^2$, $R^3$, $R^4$ are as defined in claim 1 or a salt or an ester thereof.

**12.** A process according to claim 11. wherein the mediator is 1-hydroxybenzotriazole.

**13.** A process according to any of the preceding claims, wherein the concentration of mediator in the rinse liquor is from 10 μM to 0.5 mM.

**14.** A process according to any of the preceding claims, wherein the additives are surfactants and/or water conditioning agents.

**15.** A process according to any of the preceding claims, wherein the dye or print is a reactive dye.

**16.** A system for removal of excess dye from newly manufactured printed or dyed fabric or yam, which is a multi-component system comprising

- at least one enzyme selected from the group consisting of enzymes exhibiting peroxidase activity or laccase activity,

- optionally an oxidation agent, and

- at least one mediator wherein the mediator is a compound of the general formula I:

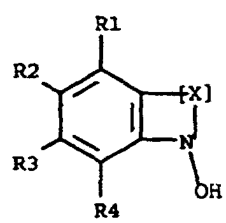

wherein $R^1$, $R^2$, $R^3$, $R^4$ are individually selected from the group consisting of hydrogen, halogen, hydroxy, formyl, carboxy and salts and esters thereof, amino, nitro, $C_1$-$C_{12}$ alkyl, $C_1$-$C_6$ alkoxy, carbonyl($C_1$-$C_{12}$ alkyl), aryl, in particular phenyl, sulfo, aminosulfonyl, carbamoyl, phosphono, phosphonooxy, and salts and esters thereof, wherein the $R^1$, $R^2$, $R^3$, $R^4$ may be substituted with $R^5$, wherein $R^5$ represents hydrogen, halogen, hydroxy, formyl, carboxy and salts and esters thereof, amino, nitro, $C_1$-$C_{12}$ alkyl, $C_1$-$C_8$ alkoxy, carbonyl($C_1$-$C_{12}$ alkyl), aryl, in particular phenyl, sulfo, aminosulfonyl, carbamoyl, phosphono, phosphonooxy, and salts and esters thereof,
[X] represents a group from (-N=N-), $(-N=CR^6-)_m$, $(-CR^6=N-)_m$, $(-CR^7=CR^8-)_m$,
and m is 1 or 2. wherein said mediator is present at a concentration of from 1 μM to 1 mM when said multi-component system is dissolved in a rinse liquor, and

- optionally additives.

**17.** A system according to claim 16, wherein the enzyme is a laccase (EC 1.10.3.2), a catechol oxidase (EC 1.10.3.1). a bilirubin oxidase (EC 1.3.3.5), a peroxidase (EC 1.11.1.7), or a haloperoxidase, such as a chloride peroxidase (EC 1.11.1.10) or any fragment derived therefrom exhibiting enzymatic activity or synthetic or semisynthetic derivatives.

**18.** A system according to claim 17, wherein the peroxidase is derived from a strain of *Coprinus* or from soybean.

**19.** A system according to claim 17, wherein the laccase is derived from a strain of Fomes, *Trametes, Rhizoctonia, Coprinus, Myceliophthora, Schytalidium,* or *Polyporus*.

**20.** A system according to any of the claims 16-19, wherein the oxidation agent is a $H_2O_2$ source.

**21.** A system according to claim 20, wherein the $H_2O_2$ source is hydrogen peroxide, a perborate, a percarbonate, a persulfate, a peroxycarboxylic acid or a salt thereof, or an enzymatic system capable of generating hydrogen peroxide.

**22.** A system according to claim 21, wherein the concentration of $H_2O_2$ is from 0.01 to 50 mM, preferably 0.1 to 5 mM.

**23.** A system according to any of the claims 16-19. wherein the oxidation agent is a $O_2$ source.

**24.** A system according to claim 23, wherein the $O_2$ source is air, pure $O_2$, or an $O_2$ generating enzymatic system.

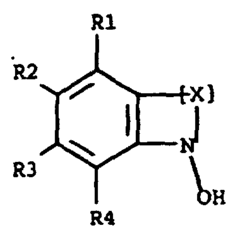

**25.** A system according to claim 16, wherein the mediator is a compound of the general formula II:

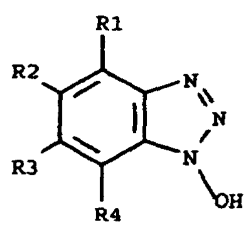

wherein $R^1$, $R^2$, $R^3$, $R^4$ are as defined in claim 16 or a salt or ester thereof.

**26.** A system according to claim 25, wherein the mediator is 1-hydroxybenzotriazole.

**27.** A system according to any of the claims 16-26, wherein one component is a solution comprising the enzyme(s) and the mediator(s).

**28.** A system according to any of the claims 16-26, wherein one component is a slurry comprising the enzyme(s) and the mediator(s).

**29.** A system according to any of the claims 16-26, wherein one component is a granulate comprising the enzyme(s) and the mediator(s).

**30.** A system according to claim 16-26, wherein one component is a granulate comprising the enzyme(s) and another component is a granulate comprising the mediator(s).

**31.** Use of components comprising

- at least one enzyme selected from the group consisting of enzymes exhibiting peroxidase activity or laccase

activity,

- optionally an oxidation agent, and
- at least one mediator wherein the mediator is a compound of the general formula I:

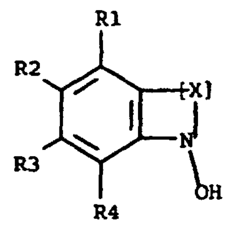

wherein $R^1$, $R^2$, $R^3$. $R^4$ are individually selected from the group consisting of hydrogen, halogen, hydroxy, formyl, carboxy and salts and esters thereof, amino, nitro, $C_1$-$C_{12}$ alkyl, $C_1$-$C_8$ alkoxy, carbonyl($C_1$-$C_{12}$ alkyl), aryl, in particular phenyl, sulfo, aminosulfonyl, carbamoyl, phosphono, phosphonooxy, and salts and esters thereof, wherein the R', $R^2$, $R^3$, $R^4$ may be substituted with $R^5$, wherein $R^5$ represents hydrogen, halogen, hydroxy, formyl, carboxy and salts and esters thereof, amino, nitro, $C_1$-$C_{12}$ alkyl, $C_1$-$C_8$ alkoxy, carbonyl($C_1$-$C_{12}$ alkyl), aryl, in particular phenyl, sutfo, aminosulfonyl, carbamoyl. phosphono, phosphonooxy, and salts and esters thereof,
[X] represents a group from (-N=N-), $(-N=CR^6-)_m$, $(-CR^6=N-)_m$, $(-CR^7=CR^8-)_m$,
and m is 1 or 2, and

- optionally additives

for the preparation of a multi-component system for removal of excess dye or print from newly manufactured fabric or yam.

**Patentansprüche**

1. Verfahren zur Entfernung von überschüssigem Farbstoff aus neu hergestelltem bedrucktem oder gefärbtem Stoff oder Garn, umfassend die Behandlung mit einer Spüllauge, umfassend

- mindestens ein Enzym, ausgewählt aus der Gruppe, bestehend aus Enzymen, die Peroxidaseaktivität oder Laccascaktivität zeigen,
- ein Oxidationsmittel und
- mindestens einen Vermittler. wobei der Vermittler eine Verbindung der allgemeinen Formel I ist:

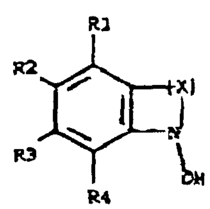

wobei $R^1$, $R^2$, $R^3$, $R^4$ einzeln ausgewählt sind aus der Gruppe, bestehend aus Wasserstoff, Halogen, Hydroxy, Formyl, Carboxy und Salzen und Estern davon, Amino, Nitro, $C_1$-$C_{12}$-Alkyl, $C_1$-$C_6$-Alkoxy, Carbonyl($C_1$-$C_{12}$-alkyl), Aryl, insbesondere Phenyl, Sulfo, Aminosutfbnyl, Carbamoyl, Phosphon, Phosphonoxy und Salzen und Estern davon, wobei $R^1$, $R^2$, $R^3$, $R^4$ mit $R^5$ substituiert sein können, wobei $R^5$ Wasserstoff, Halogen, Hydroxy, Formyl, Carboxy und Salze und Ester davon, Amino. Nitro, $C_1$-$C_{12}$-Alkyl, $C_1$-$C_6$-Alkoxy, Carbonyl($C_1$-$C_{12}$-alkyl), Aryl, insbesondere Phenyl, Sulfo, Aminosulfonyl, Carbamoyl, Phosphon, Phosphonoxy und Salze und Ester davon darstellt,
[X] eine Gruppe aus (-N=N-), $(-N=CR^6-)_m$, $(-CR^6=N-)_m$, $(-CR^7=CR^8-)_m$ darstellt,

und m 1 oder 2 ist, wobei der Vermittler in der Spüllauge mit einer Konzentration von 1 M bis 1 mM vorliegt, und

- gegebenenfalls Zusätze.

**2.** Verfahren nach Anspruch I, wobei das Enzym eine Laccase (EC 1.10.3.2), eine Catechotoxidase (EC 1.10.3.1), eine Bilirubinoxidase (EC 1.3.3.5), eine Peroxidase (EC 1.11.1.7) oder eine Halogenperoxidasc wie eine Chloridperoxidase (EC 1.11.1.10) oder ein beliebiges davon abgelcitetes Fragment, das enzymatische Aktivität zeigt, oder synthetische oder halbsynthetische Derivate davon ist.

**3.** Verfahren nach Anspruch 2, wobei die Peroxidase von einem Stamm von *Coprinus* oder von Sojabohne abgeleitet ist.

**4.** Verfahren nach Anspruch 2, wobei die Laccasc von einem Stamm von *Fomes, Trametes*, *Rhizoctonia, Coprinus, Myceliophthora, Schytalidium* oder *Polyporus* abgeleitet ist.

**5.** Verfahren nach einem der vorangehenden Ansprüche, wobei die Enzymmenge 0,005 bis 5 mg Enzymprotein pro Liter Spüllauge, vorzugsweise 0,02 bis 2 mg Enzymprotein pro Liter Spüllauge, stärker bevorzugt 0,05 bis 1 mg Enzymprotein pro Liter Spüllauge beträgt.

**6.** Verfahren nach einem der vorangehenden Ansprüche, wobei das Oxidationsmittel eine $H_2O_2$-Quelle ist.

**7.** Verfahren nach Anspruch 6, wobei die $H_2O_2$-Quelle Wasserstoffperoxid, ein Perborat, ein Percarbonat, ein Persulfat, eine Peroxycarbonsäure oder ein Salz davon oder ein enzymatisches System, das Wasserstuffperoxid bilden kann, ist.

**8.** Verfahren nach Anspruch 7, wobei die $H_2O_2$-Kanzentration 0,01 bis 50 mM, vorzugsweise 0,1 bis 5 mM beträgt.

**9.** Verfahren nach einem der Ansprüche 1-5, wobei das Oxidationsmittel eine $O_2$-Quelle ist.

**10.** Verfahren nach Anspruch 9, wobei die $O_2$-Quelle Luft, reiner $O_2$ oder ein $O_2$ bildendes enzymatisches System ist.

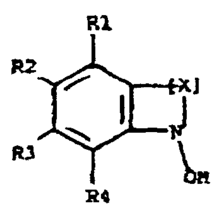

**11.** Verfahren nach Anspruch 1, wobei der Vermittler eine Verbindung der allgemeinen Formel II:

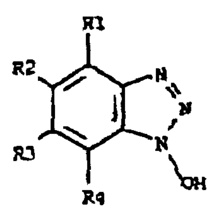

wobci $R^1$, $R^2$, $R^3$, $R^4$ wie in Anspruch 1 definiert sind, oder cin Satz davon ist.

**12.** Verfahren nach Aaspnich 11, wobei der Vermittler 1-Hydroxybenzotriazol ist.

**13.** Verfahren nach einem der vorangehenden Ansprüchc, wobei die Vennittlerkonzentration in der Spüllauge 10 µM bis 0,5 mM beträgt.

**14.** Verfahren nach einem der vorangehenden Ansprüche, wobei die Zusätze oberflächenaktive Mittel und/oder Wasserkonditionierungsmittel sind.

**15.** Verfahren nach einem der vorangehenden Ansprüche. wobei der Farbstoff oder Aufdruck ein Reaktivfarbstoff ist.

**16.** System zur Entfernung von überschüssigem Farbstoff aus neu hergestelltem bedrucktem oder gefärbtem Stoff oder Garn, wobei es sich um ein Mehrkomponentensystem handelt, umfassend:

- mindestens ein Enzym, ausgewählt aus der Gruppe, bestehend aus Enzymen, die Peroxidaseaktivität oder Laccasealctivität zeigen,
- gegebenenfalls ein Oxidationsmittel und
- mindestens einen Vermittler, wobei der Vermittler eine Verbindung der allgemeinen Formel I ist:

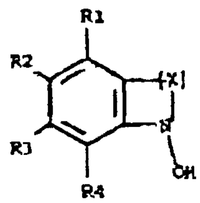

wobei $R^1$, $R^2$, $R^3$, $R^4$ einzeln ausgewählt sind aus der Gruppe, bestehend aus Wasserstoff, Halogen, Hydroxy, Formyl, Carboxy und Salzen und Estern davon, Amino, Nitro, $C_1$-$C_{12}$-Alkyl, $C_1$-$C_6$-Alkoxy, Carbonyl($C_1$-$C_{12}$-alkyl), Aryl, insbesondere Phenyl, Sulfo, Aminosulfonyl, Carbamoyl, Phosphon, Phosphonoxy und Salzen und Estern davon, wobei $R^1$, $R^2$, $R^3$, $R^4$ mit $R^5$ substituiert scin können, wobei $R^5$ Wasserstoff, Halogen, llydroxy, Formyl, Carboxy und Salze und Ester davon, Amino, Nitro, $C_1$-$C_{12}$-Alkyl, $C_1$-$C_6$-Alkoxy, Carbonyl($C_1$-$C_{12}$-alkyl), Aryl, insbesondere Phenyl, Sulfo, Atninosulfonyl, Carbamoyl, Phosphon, Phosphonoxy und Salze und Ester davon darstellt,
[X] eine Gruppe aus (-N=N-), $(-N=CR^6-)_m$, $(-CR^6=N-)_m$, $(-CR^7-CR^8-)_m$ darstellt,
und m 1 oder 2 ist, wobei der Vermittler in der Spüllauge mit einer Konzentration von 1 M bis 1 mM vorliegt, wenn das Mehrkomponentensystem in einer Spüllauge gelöst ist, und

- gegebenenfalls Zusätze.

**17.** System nach Anspruch 16, wobei das Enzym eine Laccase (EC 1.10.3.2), eine Catecholoxidase (EC 1.10.3.1), eine Bilirubinoxidase (EC 1.3.3.5), eine Peroxidase (EC 1.11.1.7) oder eine Halogenperoxidase wie eine Chioridperoxidase (EC 1.11.1.10) oder ein beliebiges davon abgeleitetes Fragment, das enzymatische Aktivität zeigt, oder synthetische oder halbsynthetische Derivate davon ist.

**18.** System nach Anspruch 17, wobei die peroxidase von einem Stamm von *Coprinus* oder von Sojabohne abgeleitet ist.

**19.** System nach Anspruch 17, wobei die Laccase von einem Stamm von *Fomes, Trumetes, Rhizocronia*, *Coprinus, Myceliophthora, Schytalidium* oder *Polypurus* abgeleitet ist.

**20.** System nach einem der Ansprüche 16-19, wobei das Oxidationsmittel eine $H_2O_2$-Quelle ist.

**21.** System nach Anspruch 20, wobei die $H_2O_2$-Quelle Wasserstoffperoxid, ein Perborat, ein Percarbonat, ein Persulfat, eine Peroxycarbonsäure oder ein Salz davon oder ein cnzymatisches System, das Wasserstoffperoxid bilden kann, ist.

**22.** System nach Anspruch 21, wobei die $H_2O_2$-Konzentration 0,01 bis 50 mM, vorzugsweise 0,1 bis 5 mM beträgt.

**23.** System nach einem der Ansprüche 16-19, wobci das Oxidationsmittel eine $O_2$-Quelle ist.

**24.** System nach Anspruch 23, wobei die $O_2$-Quelle Luft, reiner $O_2$ oder ein $O_2$ bildendes enzymatisches System ist.

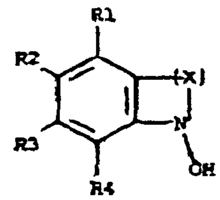

**25.** System nach Anspruch 16, wobei der vermittler eine Verbindung der allgemeinen Formel II:

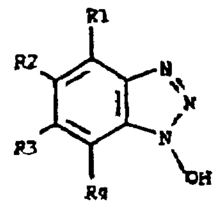

wobei $R^1$, $R^2$, $R^3$, $R^4$ wie in Anspruch 1 definiert sind, oder ein Salz davon ist.

**26.** System nach Anspruch 25, wobei der Vermittler 1-Hydroxybeniouriazol ist.

**27.** System nach einem der Ansprüche 16-26, wobei ein Bestandteil eine Lösung, umfassend das (die) Enzyme und den (die) Vermittler, ist.

**28.** System nach einem der Ansprüche 16-26, wobei ein Bestandteil eine Aufschlämmung, umfassend das (die) Enzyme und den (die) Vermittler, ist.

**29.** System nach einem der Ansprüche 16-26, wobei ein Bestandteil ein Granulat, umfassend das (die) Enzyme und den (die) Vermittler, ist.

**30.** System nach einem der Ansprüche 16-26, wobei ein Bestandteil ein Granulat, umfassend das (die) Enzyme und ein anderer Bestandteil ein Granulat, umfassend den (die) Vermittler, ist.

**31.** Verwendung von Bestandteilen, umfassend:

- mindestens ein Enzym, ausgewählt aus der Gruppe, bestehend aus Enzymen, die Peroxidaseaktivität oder Laccaseaktivität zeigen,
- gegebenenfalls ein Oxidationsmittel und
- mindestens einen Vermittler, wobei der Vermittler eine Verbindung der allgemeinen Formel 1 ist:

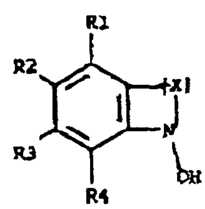

wobei $R^1$, $R^2$, $R^3$, $R^4$ einzeln ausgewählt sind aus der Gruppe, bestehend aus Wasserstoff, Halogen, Hydroxy, Formyl, Carboxy und Salzen und Estern davon, Amino, Nitro, $C_1$-$C_{12}$-Alkyl, $C_1$-$C_6$-Alkoxy, Carbonyl($C_1$-$C_{12}$-alkyl), Aryl, insbesondere Phenyl, Sulfo, Aminosulfonyl, Carbamoyl, Phosphon, Phosphonoxy und Salzen und Estern davon, wobei $R^1$, $R^2$, $R^3$, $R^4$ mit $R^5$ substituiert sein können, wobei $R^5$ Wasscrstoff, Halogen, llydroxy, Formyl, Carboxy und Salze und Ester davon. Amino, Nitro, $C_1$-$C_{12}$-Alkyl, $G_1$-$C_6$-Alkaxy, Carbonyl($C_1$-$C_{12}$-alkyl), Aryl, insbesondere Phenyl, Sulfo, Aminosulfonyl, Carbamoyl, Phosphon, Phosphonoxy und Salze und Ester davon darstellt,

[X] eine Gruppe aus (-N=N-), $(-N=CR^6-)_m$, $(-CR^6=N-)_m$, $(-CR^7=CR^8-)_m$ darstellt, und m 1 oder 2 ist, und

- gegebenenfalls Zusätze.

zur Herstellung eines Mehrkomponentensystems zur Entfernung von überschüssigem Farbstoff oder Aufdruck aus neu hergestelhem Stoff odcr Garn.

## Revendications

**1.** Un procédé d'élimination de l'excédent de teinture d'un tissu imprimé ou teint nouvellement fabriqué ou d'un fil, comprenant le traitement avec une liqueur de rinçage comportant :

- au moins une enzyme choisie dans le groupe comprenant les enzymes présentant une activité peroxydase ou une activité laccase,
- un agent d'oxydation, et
- au moins un médiateur dans lequel le médiateur est un composé de formule générale I :

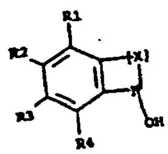

dans laquelle $R^1$, $R^2$, $R^3$, $R^4$ sont choisis individuellement à partir du groupe comprenant l'hydrogène, un halogène, un radical hydroxy, formyle, carboxy et leurs sels et esters, amino, nitro, alkyle en $C_1$-$C_{12}$, alcoxy en $C_1$-$C_6$, carbonyl (alkyle en $C_1$-$C_{12}$), aryle, en particulier phényle, sulfo, aminosulfonyle, carbamoyle, phosphono, phosphonooxy et leurs sels et esters, dans laquelle $R^1$, $R^2$, $R^3$, $R^4$ peuvent être substitués avec $R^5$, dans laquelle $R^5$ représente de l'hydrogène, un halogène, un radical hydroxy, formyle, carboxy et leurs sels et esters, amino, nitro, alkyle en $C_1$-$C_{12}$, alcoxy en $C_1$-$C_6$, carbonyl (alkyle en $C_1$-$C_{12}$), aryle, en particulier phényle, sulfo, aminosulfonyle, carbamoyle, phosphono, phosphonooxy et leurs sels et esters,

[X] représente un groupe choisi parmi (-N = N-), $(-N = CR^6-)_m$ $(-CR^6 = N-)_m$ $(-CR^7 = CR^6-)$m,

et m est 1 ou 2, dans lequel ledit médiateur est présent dans ladite liqueur de rinçage à une concentration de 1 µM à 1 mM, et

- des additifs facultatifs.

**2.** Un procédé selon la revendication 1, dans lequel l'enzyme est une laccase (EC 1.10.3.2), une catéchol oxydase (EC 1.10.3.1), une bilirubine oxydase (EC 1.3.3.5), une peroxydase (EC 1.11.1.7) ou une haloperoxydase comme une chlorure peroxydase (EC 1.11.1.10) ou n'importe quel fragment dérivé de celle-ci présentant une activité enzymatique ou leurs dérivés synthétiques ou semi-synthétiques.

**3.** Un procédé selon la revendication 2, dans lequel la peroxydase dérive d'une souche de Coprinus ou de graine de soja.

**4.** Un procédé selon la revendication 2, dans lequel la laccase dérive d'une souche de Fomes, Trametes, Rhizoctonia, Coprinus, Myceliophthora, Schytalidium ou Polyporus.

**5.** Un procédé selon l'une quelconque des revendications précédentes, dans lequel la quantité d'enzyme est de 0,005 à 5 mg d'enzyme protéine pour 1 1 de liqueur de rinçage, de préférence de 0,02 à 2 mg d'enzyme protéine pour 1 1 de liqueur de rinçage, mieux encore de 0,05 à 1 mg d'enzyme protéine pour 1 1 de liqueur de rinçage.

**6.** Un procédé selon l'une quelconque des revendications précédentes, dans lequel l'agent d'oxydation est une source de $H_2O_2$.

**7.** Un procédé selon la revendication 6, dans lequel la source de $H_2O_2$ est du peroxyde d'hydrogène, un perborate,

un percarbonate, un persulfate, un acide peroxycarboxylique ou un sel de celui-ci ou un système enzymatique capable de générer du peroxyde d'hydrogène.

**8.** Un procédé selon la revendication 7, dans lequel la concentration de $H_2O_2$ est de 0,01 à 50 mM, de préférence de 0,1 à 5 mM.

**9.** Un procédé selon l'une quelconque des revendications 1 à 5, dans lequel l'agent d'oxydation est une source de $O_2$.

**10.** Un procédé selon la revendication 9, dans lequel la source de $O_2$ est de l'air, du $O_2$ pur ou un système enzymatique générant du $O_2$.

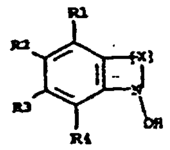

**11.** Un procédé selon la revendication 1, dans lequel le médiateur est un composé de formule générale II :

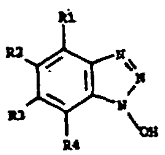

dans laquelle $R^1$, $R^2$, $R^3$, $R^4$ sont comme défini dans la revendication 1 ou un sel ou un ester de celui-ci.

**12.** Un procédé selon la revendication 11, dans lequel le médiateur est le 1-hydroxybenzotriazole.

**13.** Un procédé selon l'une quelconque des revendications précédentes, dans lequel la concentration de médiateur dans la liqueur de rinçage est de 10 μM à 0,5 mM.

**14.** Un procédé selon l'une quelconque des revendications précédentes, dans lequel les additifs sont des tensioactifs et/ou des agents de conditionnement d'eau.

**15.** Un procédé selon l'une quelconque des revendications précédentes, dans lequel la teinture ou l'impression est une teinture réactive.

**16.** Un système d'élimination de l'excédent de teinture d'un tissu imprimé ou teint nouvellement fabriqué ou d'un fil, qui est un système multicomposants comprenant :

- au moins une enzyme choisie dans le groupe comprenant les enzymes présentant une activité peroxydase ou une activité laccase,
- éventuellement un agent d'oxydation, et
- au moins un médiateur dans lequel le médiateur est un composé de formule générale I :

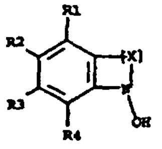

dans laquelle $R^1$, $R^2$, $R^3$, $R^4$ sont choisis individuellement à partir du groupe comprenant l'hydrogène, un halogène, un radical hydroxy, formyle, carboxy et leurs sels et esters, amino, nitro, alkyle en $C_1$-$C_{12}$, alcoxy en $C_1$-$C_6$, carbonyl (alkyle en $C_1$-$C_{12}$), aryle, en particulier phényle, sulfo, aminosulfonyle, carbamoyle, phosphono,

phosphonooxy et leurs sels et esters, dans laquelle $R^1$, $R^2$, $R^3$, $R^4$ peuvent être substitués avec $R^5$, dans laquelle $R^5$ représente de l'hydrogène, un halogène, un radical hydroxy, formyle, carboxy et leurs sels et esters, amino, nitro, alkyle en $C_1$-$C_{12}$, alcoxy en $C_1$-$C_6$, carbonyl (alkyle en $C_1$-$C_{12}$), aryle, en particulier phényle, sulfo, aminosulfonyle, carbamoyle, phosphono, phosphonooxy et leurs sels et esters,

[X] représente un groupe choisi parmi (-N = N-), (-N = $CR^6$-)$_m$ (-$CR^6$ = N-)$_m$ (-$CR^7$ = $CR^6$-)m,

et m est 1 ou 2, dans lequel ledit médiateur est présent selon une concentration de 1 µM à 1 mM lorsque ledit système multicomposants est dissous dans une liqueur de rinçage, et

- des additifs facultatifs.

**17.** Un système selon la revendication 16, dans lequel l'enzyme est une laccase (EC 1.10.3.2), une catéchol oxydase (EC 1.10.3.1), une bilirubine oxydase (EC 1.3.3.5), une peroxydase (EC 1.11.1.7) ou une haloperoxydase comme une chlorure peroxydase (EC 1.11.1.10) ou n'importe quel fragment dérivé de celle-ci présentant une activité enzymatique ou leurs dérivés synthétiques ou semi-synthétiques.

**18.** Un système selon la revendication 17, dans lequel la peroxydase dérive d'une souche de Coprinus ou de graine de soja.

**19.** Un système selon la revendication 17, dans lequel la laccase dérive d'une souche de Fomes, Trametes, Rhizoctonia, Coprinus, Myceliophthora, Schytalidium ou Polyporus.

**20.** Un système selon l'une quelconque des revendications 16 à 19, dans lequel l'agent d'oxydation est une source de $H_2O_2$.

**21.** Un système selon la revendication 20, dans lequel la source de $H_2O_2$ est du peroxyde d'hydrogène, un perborate, un percarbonate, un persulfate, un acide peroxycarboxylique ou un sel de celui-ci ou un système enzymatique capable de générer du peroxyde d'hydrogène.

**22.** Un système selon la revendication 21, dans lequel la concentration de $H_2O_2$ est de 0,01 à 50 mM, de préférence de 0,1 à 5 mM.

**23.** Un système selon l'une quelconque des revendications 16 à 19, dans lequel l'agent d'oxydation est une source de $O_2$.

**24.** Un système selon la revendication 23, dans lequel la source de $O_2$ est de l'air, du $O_2$ pur ou un système enzymatique générant du $O_2$.

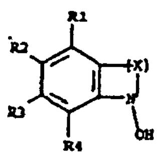

**25.** Un système selon la revendication 16, dans lequel le médiateur est un composé de formule générale II :

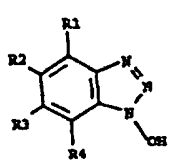

dans laquelle $R^1$, $R^2$, $R^3$, $R^4$ sont comme défini dans la revendication 16 ou un sel ou un ester de celui-ci.

**26.** Un système selon la revendication 25, dans lequel le médiateur est le 1-hydroxybenzotriazole.

**27.** Un système selon l'une quelconque des revendications 16 à 26, dans lequel un composant est une solution comprenant la/les enzyme(s) et le/les médiateur(s).

**28.** Un système selon l'une quelconque des revendications 16 à 26, dans lequel un composant est une suspension comprenant la/les enzyme(s) et le/les médiateur(s).

**29.** Un système selon l'une quelconque des revendications 16 à 26, dans lequel un composant est un granulat comprenant la/les enzyme(s) et le/les médiateur(s).

**30.** Un système selon l'une quelconque des revendications 16 à 26, dans lequel un composant est un granulat comprenant la/les enzyme(s) et un autre composant est un granulat comprenant le/les médiateur(s).

**31.** Utilisation de composants renfermant :

- au moins une enzyme choisie dans le groupe comprenant les enzymes présentant une activité peroxydase ou une activité laccase,
- facultativement un agent d'oxydation, et
- au moins un médiateur dans lequel le médiateur est un composé de formule générale I :

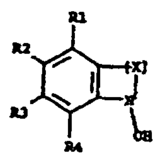

dans laquelle $R^1$, $R^2$, $R^3$, $R^4$ sont choisis individuellement à partir du groupe comprenant l'hydrogène, un halogène, un radical hydroxy, formyle, carboxy et leurs sels et esters, amino, nitro, alkyle en $C_1$-$C_{12}$, alcoxy en $C_1$-$C_6$, carbonyl (alkyle en $C_1$-$C_{12}$), aryle, en particulier phényle, sulfo, aminosulfonyle, carbamoyle, phosphono, phosphonooxy et leurs sels et esters, dans laquelle $R^1$, $R^2$, $R^3$, $R^4$ peuvent être substitués avec $R^5$, dans laquelle $R^5$ représente de l'hydrogène, un halogène, un radical hydroxy, formyle, carboxy et leurs sels et esters, amino, nitro, alkyle en $C_1$-$C_{12}$, alcoxy en $C_1$-$C_6$, carbonyl (alkyle en $C_1$-$C_{12}$), aryle, en particulier phényle, sulfo, aminosulfonyle, carbamoyle, phosphono, phosphonooxy et leurs sels et esters,

[X] représente un groupe choisi parmi (-N = N-), $(-N = CR^6-)_m$ $(-CR^6 = N-)_m$ $(-CR^7 = CR^6-)$m,
et m est 1 ou 2, et

- des additifs facultatifs

pour la préparation d'un système multicomposants pour l'élimination d'excédent de teinture ou d'impression d'un tissu ou fil nouvellement fabriqué.